# EUROPEAN PATENT SPECIFICATION

(11) **EP 3 315 086 B1**
(45) Date of publication and mention of the grant of the patent: **17.06.2020**
(21) Application number: 16196472.1
(22) Date of filing: 28.10.2016
(51) Int. Cl.: A61B 5/00, A61B 5/042, A61B 18/14, A61B 18/00, A61B 17/00

(54) **ELONGATED MEDICAL DEVICE SUITABLE FOR INTRAVASCULAR INSERTION AND METHOD OF MAKING SUCH A DEVICE**
LÄNGLICHE MEDIZINISCHE VORRICHTUNG ZUM INTRAVASKULÄREN EINSETZEN UND VERFAHREN ZUR HERSTELLUNG SOLCH EINER VORRICHTUNG
DISPOSITIF MÉDICAL ALLONGÉ CONÇU POUR UNE INSERTION INTRAVASCULAIRE ET PROCÉDÉ DE FABRICATION D'UN TEL DISPOSITIF

(43) Date of publication of application: 02.05.2018
(73) Proprietor: Ablacon Inc., Wheat Ridge, CO 80033 (US)
(72) Inventor: Ruppersberg, Peter, 1807 Blonay (CH)
(74) Representative: Betten & Resch

(56) References cited:
- EP-A1- 1 056 403
- US-A1- 2012 271 139
- US-A1- 2014 257 069
- US-A1- 2015 105 645

## Description

The present invention relates generally to elongated medical devices suitable for intravascular insertion, comprising a flexible elongated body having a distal portion with a distal end and a proximal portion, and an electrode assembly located at the distal portion. The electrode assembly comprises a plurality of at least x=4 support arms, wherein x is evenly divisible by the number 2. Each support arm has a proximal end part, a distal end part and a main part between the proximal end part and the distal end part. The plurality of at least x=4 support arms comprises first and second neighboring support arms that form pairs of neighboring support arms. The electrode assembly further comprises a plurality of electrodes arranged on the support arms for collecting electrophysiological data. The plurality of at least x=4 support arms being configured to have a first condition, where the plurality of at least x=4 support arms is arranged in a bundle, and a second condition, where the plurality of at least x=4 support arms is expanded, forming a basket type structure.

Such elongated medical devices suitable for intravascular insertion may be manually or robotically steerable catheters for the exploration or treatment of vessels or organs or other body cavities or guide wires for guiding catheters or the like medical apparatuses. The present invention further relates to a method of making such an elongated medical device with the features of the independent method claim.

The present invention especially relates to an elongated medical device suitable for intravascular insertion with individual features of claim 1. The present invention further relates to a method of making an elongated medical device suitable for intravascular insertion with individual features of claim 12.

Elongated medical devices suitable for intravascular insertion, such as catheters, especially ablation catheters, and guide wires for guiding catheters through vessels, organs or other body cavities are e.g. used in the treatment of atrial fibrillation (Afib). Atrial fibrillation is the most frequent arrhythmic disorder of the heart. Blood clotting occurring in the fibrillating atria is one main cause of stroke. Afib is also one of the most important disorders associated with a high risk of fatality. The cause of Afib has been subject to intensive scientific investigations and is largely understood. In most patients, the pulmonary veins draining into the left atrium are the sources of rapid arrhythmic action potentials which trigger circular excitation patterns (rotors), in the left atrium that induce a high frequency fibrillation through their re-entry mechanism. Those rotors have the character of small action potential cyclones of 2 to 3 cm² in size. The likelihood of occurrence of those rotors and the frequency of pathological action potential generation in the pulmonary veins increases with fibrotic structural changes and certain modifications of ion channel expression patterns in atrial cells with age.

The only potentially curative treatments for Afib are open heart surgery or cardiac ablation employing a catheter for those parts of the atrial wall tissue which originate, transmit or maintain the pathologic excitation circles.

Open heart surgery and catheter ablation are limited by potentially fatal and/or severe side effects associated with either procedure: When the integrity of the atrial wall is destroyed by excessive ablation, perforations of the atrial wall into the pericardium or fistulas into the esophagus can result in severe to deadly outcomes. The alteration of endocardial cells on the intra-cardiac surfaces can also initiate clotting in the treated atrium, which may lead to deadly strokes. That is why ablation procedures require the use of anticoagulation techniques. Last but not least, if the intensity of the ablation is kept too low to avoid the foregoing side effects, in many cases the therapeutic effect is insufficient and patients are often provided with success rates of only 50-70%.

To improve the situation, mapping catheters are often used first to identify circular excitation patterns (rotors) in the left atrium. After rotors have been identified, force sensing catheters are used that allow improved control of cardiac ablation catheter positions and pressures, which permits the intensity of tissue ablation to be better modulated and controlled. Further, water irrigation is often employed to keep endothelial tissue free of lesions during the ablation procedure, and micro-calorimetric sensors may be employed to measure and control the amount of heat delivered to the tissue during the ablation procedure.

US 8,364,234 discloses a system for sensing multiple local electric voltages from endocardial surface of a heart. The system includes a first elongate tubular member; a basket assembly having a plurality of flexible splines for guiding a plurality of exposed electrodes, the splines having proximal portions, distal portions and medial portions therein between; a proximal anchor for securely affixing the proximal portions of the splines; the proximal anchor being secured at the distal end of the first elongate tubular member; a distal tip consisting essentially of means for only securely affixing the distal portions of the splines wherein at least some of the splines in the radially expanded non-spherical shape contain a distal outward bend disposed at the distal portion of the spline at a location near to the distal tip of the basket assembly to bend the splines back towards the proximal anchor. A disadvantage of this type of mapping system is the low resolution provided by the mapping electrode array, as its splines tend to bunch or cluster when contacting a body surface, thus reducing the effective number of electrodes.

Laughner et. al. in JACC, CLINICAL ELECTROPHYSIOLOGY, 2016; 2(1):55-65. doi: 10.1016, conclude that known mapping basket catheters (MBC's) provide insufficient spatial resolution due to poor contact, demonstrate frequent bunching of basket splines, and possess inadequate electrode density to permit accurate detection of rotors near the equatorial electrodes of MBCs.

US 2015 105645 A1 discloses a mapping device for mapping a tissue surface including an elongate shaft and an electrode assembly. The electrode assembly includes a plurality of splines and a plurality of electrodes disposed on at least some of the splines. The electrode assembly is capable of moving between a collapsed configuration and an expanded configuration. In the expanded configuration, the electrode assembly may have a generally planar structure.

It is hence an aim or object of the present invention to provide an elongated medical device suitable for intravascular insertion that avoids the disadvantages of the prior art and which is more stable when in contact with a body surface.

The invention is defined in appended independent claims 1 and 10. Preferred embodiments are defined in the dependent claims.

These and other objects of the present invention are accomplished by providing an elongated medical device suitable for intravascular insertion where each first and second neighboring support arms are combined at their proximal end parts to form united end parts, such that all pairs of neighboring support arms are united at their proximal end parts, and that each second and first neighboring support arms are combined at their distal end parts, to form united end parts, such that all pairs of neighboring support arms are united at their distal end parts, and wherein each support arm is combined at its distal end part with a different neighboring support arm than at its proximal end part. United end parts in this respect is to be understood as neighboring distal end parts or proximal end parts of the support arms that are connected to each other such that these neighboring end parts behave like a single end part of the neighboring support arms.

The basket structured electrode assembly according to the invention with united end parts allows for a stable basket structure that is resistant to bunching or clustering of the electrode carrying support arms when contacting a surface. Accordingly, a relative even distribution of electrodes is achieved even when the device is in operation and touches a body surface, resulting in a good spatial resolution.

In a further preferred embodiment of the invention, the combination of the proximal end parts and/or the distal end parts of neighboring support arms forming united end parts is realized by combining means that are disposed at each united end part. Combining means may comprises one or more of a ring member, an adhesive joint, a clamping element and a lug or any combination or plurality thereof. With the combining means neighboring end parts are joint with each other in an easy and efficient way.

Advantageously, in one embodiment each support arm comprises a flexible electrical polymeric substrate or sheet that carries the electrodes and the electrical conductors for connecting to the electrodes electrically, wherein the united end parts of the support arms share a common flexible electrical polymeric substrate or sheet. The flexible electrical polymeric sheet may be formed as a PCB layer or sheet. The common flexible electrical polymeric substrate, sheet or PCB layer forms the combining means of the two neighboring support arms which are formed as a quasi single arm in the end parts of the neighboring support arms. In one embodiment, the electrodes and electrical conductors formed on the flexible substrate may be formed using well-known thin film deposition and manufacturing techniques. In other embodiments, or in addition, the substrate may include electrical conductors and/or electrodes formed at least partially using electroceramic techniques.

In a preferred embodiment, the number x of support arms equals 4, 6, 8, 10 or 12. With such numbers of support arms a uniform distribution of electrodes is obtained, and at the same time, it is possible to "store" the support arms in their first condition, where the plurality of at least x=4 support arms is arranged in a bundle, within the body or tube of the elongated medical device or catheter. That is the electrode assembly with its support arms may be retracted into an inner cavity within the body or tube.

Good spatial resolution may be achieved when the plurality of electrodes comprises a number of 4 to 24 electrodes disposed on each of the support arms. E.g. for a number of x=8 support arms the resulting total number of electrodes will be in the range from 32 to 132. In general, the advantage of this arrangement is that the size of the electrode assembly and/or the number of electrodes can easily be adapted to the field and/or mode of application.

It should be mentioned though, that especially in case a higher number of support arms is used, e.g. 10 or more support arms, the numbers of electrodes on the support arm may be varied such that in a pair of neighboring support arms one support arm carries more electrodes that the other one. E.g. one support arm carries 12 electrodes and the neighboring other support arm carries 8 electrodes. With such varying numbers of electrodes, the number of electrodes in the equator area of the basket type electrode assembly could be enhanced, while the number of electrodes towards the "poles" may be reduced. In general, the advantage of this arrangement is that the size of the electrode assembly and/or the number of electrodes can easily be adapted to the field and/or mode of application.

In an alternative embodiment, the plurality of electrodes is evenly distributed on the support arms. Especially in the case of fewer support arms, e.g. in the case of 4 or 6 support arms, a good spatial resolution is obtained when the number of electrodes is evenly distributed on the support arms.

In a preferred embodiment, the basket type structure has two pole areas that lie in a basket axis, wherein in the second condition of the electrode assembly each of the support arms spans a curve or bow of about 180° between the two pole areas, and wherein an angle α defines the circumferential distance between the respective pole area and a border between the respective proximal or distal end parts and the central parts on this curve or bow, and wherein the angle α is in a range of 5° to 40°. The combining means are preferably arranged on the border and the circumferential distance of angle α to the respective pole supports the enhanced stability of the basket type structure. In case of an adhesive or welded combining means, the combining means may extend between the border and the pole area along a part of the length of the neighboring proximal and/or distal end parts of the support arms or over the entire length of the neighboring proximal and/or distal end parts.
In certain preferred embodiments the angle α may be in the range of 5° to 25°.

In a favorable embodiment, the combining means may be made of a conductive metal and are electrically connected to electric lines of the support arms such that the combining means function as mapping electrodes. Accordingly, the number of mapping electrodes and hence the spatial resolution of the medical device may efficiently be enhanced without additional efforts in terms of material and costs.

Advantageously, the medical device is formed as a catheter for the exploration or treatment of a vessel, organ or other body cavity. This catheter contains one or more of the inventive features described before.

The underlying problem is further solved by a method of making an elongated medical device, such as a cardiac mapping catheter, for electro-anatomic mapping and suitable for intravascular insertion, wherein the method comprises forming a flexible elongated body having a distal portion with a distal end and a proximal portion, and forming an electrode assembly configured to be located at or near the distal portion, the electrode assembly comprising a plurality x of at least 4 support arms, wherein x is evenly divisible by the number 2, each support arm having a proximal end part, a distal end part, and a main part located between the proximal end part and the distal end part, each of the plurality of support arms comprising a plurality of electrodes, the electrodes being configured to acquire electrophysiological signals, the plurality of support arms being configured to have a first retracted condition (UC) where the plurality of support arms is arranged in a collapsed bundle, and a second expanded condition where the plurality of support arms forms an expanded basket structure. The method further comprises that the plurality of support arms further comprises first and second neighboring support arms wherein the proximal end parts of the first and second neighboring support arms are combined or held together near or adjoining one another, the distal end part of the first support arm and are further combined or held together near or adjoining a distal end part of a neighboring support arm that is not the second support arm, the distal end part of the second support arm is combined or held together near or adjoining a distal end part of a neighboring support arm that is not the first support arm. The method further comprises that each of the plurality of support arms in the electrode assembly is combined or held together at or near its distal end part with a neighboring support arm that is different from the neighboring support arm with which it is combined or held together at its proximal end part.

Advantageously, the method comprises combining or holding together the distal end parts or proximal end parts with combining means. Preferably, the combining means comprise one or more of a clamping element, a ring member, an adhesive joint, a lug, or any combination or plurality thereof. With the combining means neighboring end parts are joint with each other in an easy and efficient way.

In a favorable embodiment of the method the basket type structure is formed to have two pole areas that lie in a basket axis, wherein in the second condition of the electrode assembly each of the support arms spans a curve or bow of about 180° between the two pole areas, and wherein an angle α defines the circumferential distance between the respective pole area and a border between the respective proximal or distal end parts and the central parts on this curve or bow, and wherein the angle α is in a range of 5° to 40°. The combining means are preferably arranged on the border and the circumferential distance of angle α to the respective pole supports the enhanced stability of the basket type structure. In case of an adhesive or welded combining means, the combining means may extend between the border and the pole area along a part of the length of the neighboring proximal and/or distal end parts of the support arms or over the entire length of the neighboring proximal and/or distal end parts. In certain preferred embodiments the angle α may be in the range of 5° to 25°. Further features of the invention, its nature and various advantages will become more apparent from the accompanying drawings and the following detailed description of the preferred embodiments, in which:
Fig. 1a is a schematic view of a basket type electro-anatomic mapping catheter according to the prior art with an electrode assembly comprising support arms, the electrode assembly being in an expanded condition with a part of the support arms touching a surface;
Fig. 2 is a schematic view of an electro-anatomic mapping system comprising an elongated medical device for exploration or treatment of a vessel or organ or other body cavity, having an electrode assembly for electro-anatomic mapping of cardiac or vessel areas, the electrode assembly being in a first, retracted condition, and a data processing and control unit / and a data output unit;
Fig. 3a is a perspective view on a distal portion of the elongated medical device according to Fig. 2 in a second, expanded condition of the electrode assembly;
Fig. 3b is a perspective view on the distal portion of an elongated medical device in a further embodiment in the second, expanded condition of the electrode assembly;
Fig. 4a is an enlarged view of an area of the electrode assembly of the elongated medical device of Fig. 3a according to the marking IVa in Fig. 3a;
Fig. 4b is an enlarged view of an area of the electrode assembly of the elongated medical device of Fig. 3b according to the marking IVb in Fig. 3b;
Fig. 4c is an enlarged view of an area of an electrode assembly of a further embodiment of an elongated medical device;
Fig. 4d is an enlarged view of an area of an electrode assembly of a further embodiment of an elongated medical device;
Fig. 5a is an enlarged cut section through a pair of neighboring support arms according to the marking Va in Fig. 4a, and
Fig. 5b is an enlarged cut section through a pair of neighboring support arms analog to Fig. 5a according to a further embodiment of an elongated medical device.

The drawings are not necessarily to scale. Like numbers refer to like parts or steps throughout the drawings.

Described herein are various embodiments of a cardiac mapping catheter, and associated components, systems and methods of making and using same.
Systems and methods configured to detect in a patient's heart a location of a source of at least one cardiac rhythm disorder are disclosed herein. In the following description, for the purposes of explanation, numerous specific details are set forth in order to provide a thorough understanding of example embodiments or aspects. It will be evident, however, to one skilled in the art that an example embodiment may be practiced without necessarily using all of the disclosed specific details.

In one embodiment an elongated medical device is provided that is suitable for intravascular insertion, such as a catheter for exploration or treatment of a vessel, organ or other body cavity which includes an electrode assembly for electro-anatomic mapping of cardiac or vessel areas or the like medical apparatus. The electrode assembly may be used to map circular excitation patterns (rotors), e.g. of the left atrium of the heart.

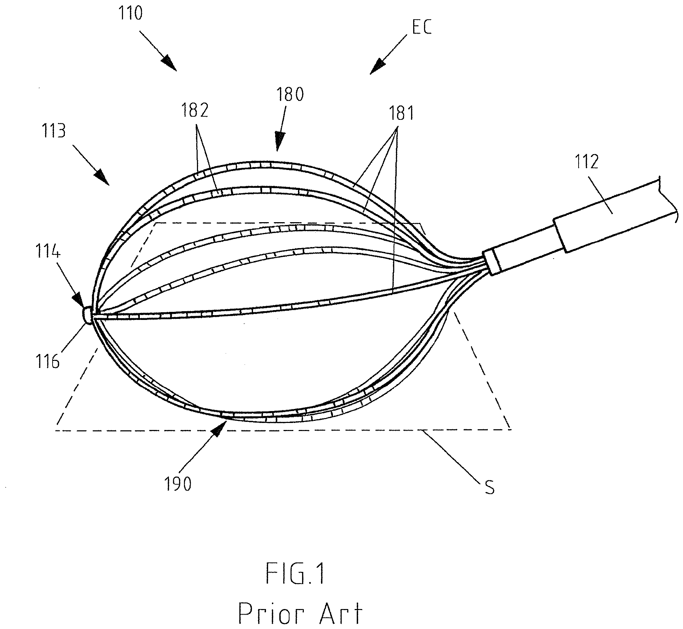

Referring now to Fig. 1, there is illustrated a prior art elongated medical device 110 which is formed as a mapping catheter. The elongated medical device 110 comprises an elongated body 112, only a distal portion 113 of which is shown in Fig. 1. The elongated medical device 110 / mapping catheter comprises a basket type electrode assembly 180 that is displayed in Fig. 1 in its expanded condition (EC). The electrode assembly 180 comprises eight support arms 181 that carry electrodes 182. In the example shown, there are eight electrodes 182 arranged on each of the eight support arms 181. A tip 116 is disposed at a distal end 114 of the distal portion 113 of the elongated medical device 110. Fig. 1 depicts the situation where the basket type electrode assembly 180 with its support arms 181 touches a surface S, e.g. a body surface. As can be seen, some of the support arms 181 bunch or accumulate on the surface S and form a cluster 190 such that the twenty four electrodes of the effected three support arms 181 effectively act as eight electrodes, as the electrodes are also bunched together forming eight clusters of each three electrodes. Due to this clustering, spatial resolution is highly reduced.

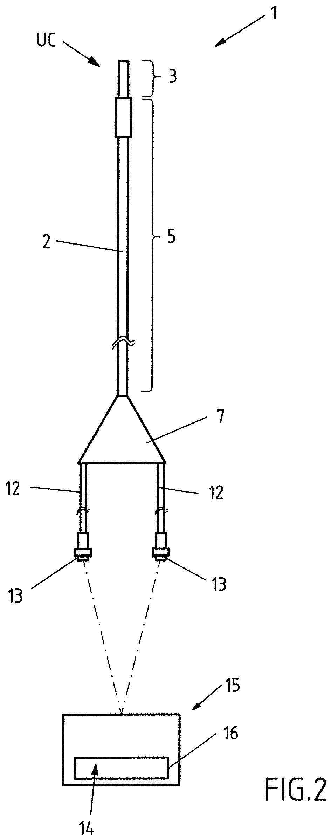

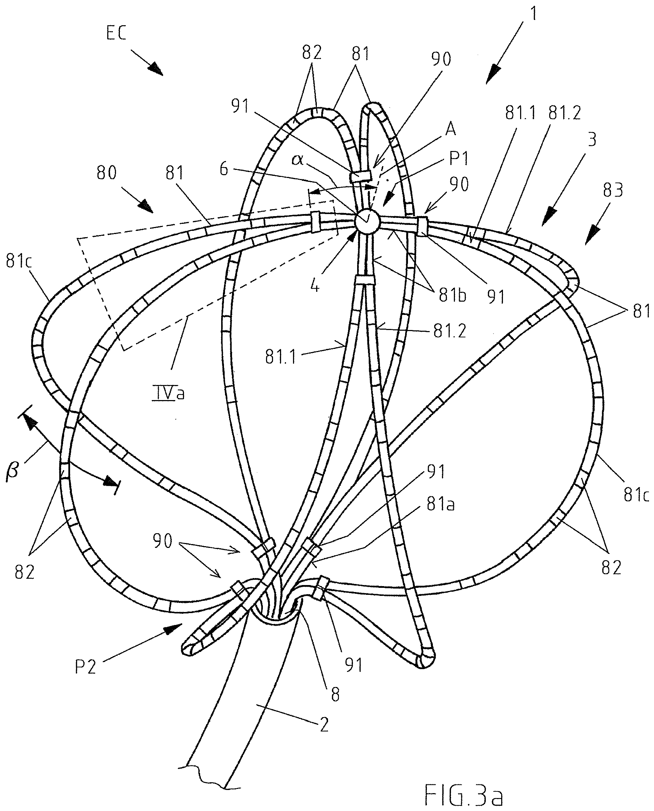

Referring to Figs. 2, 3a, 4a and 5a, there is shown an elongated medical device 1 according to the invention is formed as mapping catheter. The elongated medical device 1 comprises an elongated body 2, comprising a distal portion 3 and a proximal portion 5. At the distal portion 3 of the elongated medical device 1, there is a tip 6 arranged at the distal end 4 of the device as can especially be depicted from Fig. 3a. The elongated medical device 1 further comprises an electrode assembly 80 / mapping electrode assembly that is located at the distal portion 3 and comprises in the embodiment of Figures 2 and 3a a plurality of x = eight support arms 81 (or splines), whereby it has to be mentioned that the invention requires at least four (x = 4) such support arms 81. Each support arm 81 has a proximal end part 81a, a distal end part 81b and a main part 81c located between the proximal end part 81a and the distal end part 81b. The eight support arms 81 are connected to the tip 6 at their respective distal end parts 81b.

The central part 81c of each support arm 81 carries a plurality of electrodes 82 (also referred to as mapping electrodes), which according to one embodiment may comprise gold or gold plating for enhanced electro-conductivity; other electrically conductive metals and metal alloys are also contemplated . In one embodiment ten electrodes 82 are disposed on each support arm. In some embodiments, and by way of non-limiting example, the surface area an electrode 82 may range between about 0.01 mm² and about 0.25 mm².

A basket structure electrode assembly 80 may be formed using conventional wire, braided twisted or stranded electrical conductors and conventional electrodes. Common techniques for operably connecting metal electrodes to their corresponding wire electrical conductors in medical electrical leads include welding, swaging crimping and staking. Likewise, the flexible elongated body 2 of the catheter 1 may comprise a polymeric coating or material such as silicone, polyurethane or any other suitable polymeric material. Indeed, in one embodiment catheter 1 is formed and manufactured using largely conventional manufacturing methods and materials, where lead body 2 is formed using well-known biocompatible polymeric materials that sheath or overlie internally disposed flexible electrical conductors (e.g., braided, stranded, and/or twisted wires) that are electrically connected at their distal ends to the electrodes disposed on the support arms, and the support arms themselves are formed from similar or the same biocompatible polymeric materials, flexible electrical conductors, and metal or metal alloy electrodes.

At the proximal end of the elongated medical device 1, a handle 7 or other manipulable control device may be attached to the proximal portion 5. The handle 7 may be used to manually control expansion or retraction of the electrode assembly 80 using, for example, an internal pull wire operably connected to a proximal end of the support arms. When the internal pull wire is pushed in a distal direction, the support arms assume an expanded condition to form the basket structure. When the internal pull wire is pulled in a proximal direction, the support arms assume a retracted condition inside the elongated catheter body 2.

The electrode assembly 80 in Fig. 2 is shown in its first condition UC, where the electrode assembly 80 is stored internally in space 8 within the tubular elongated body 2 of the elongated medical device 1. In this stored position the plurality of at least x=4 support arms 81 forms a dense bundle. In the first condition UC the elongated medical device 1 / catheter may be introduced into a vessel, organ or other body cavity.

In Fig. 3a the electrode assembly 80 is in its second or expanded condition EC in which the support arms 81 project away from the elongated body 2, forming a basket type structure 83. In this second expanded condition EC the device is deployed to collect electrophysiological data, *i.e.,* for electro-anatomic mapping. As can be seen in Fig. 3a, the basket structure 83 with the support arms 81 has two pole areas P1, P2, which define a basket axis A. Between the two poles or pole areas P1, P2 each of the support arms 81 spans a curve or bow of about 180°. An angle α defines the circumferential distance between the respective pole P1 or P2 and the border between the respective proximal end parts 81a / distal end parts 81b and the central parts 81c on this curve or bow. The angle α may range between about 5° and about 40°, and between about 5° and about 25°. In the embodiment of Fig. 3a, angle α is about 15°.

It should be mentioned that the number of electrodes on the support arm may be varied such that in a pair of neighboring support arms one support arm carries more electrodes than the other one. For example, one support arm may carry twelve electrodes while a neighboring support arm carries eight electrodes. With such varying numbers of electrodes, the number of electrodes in the equator area or central portion of the basket type electrode assembly could be enhanced, while the number of electrodes towards the "poles" (which often produce the least useful information) may be reduced.

Referring to Figs. 3a, 4a and 5a, the plurality x of eight support arms 81 is shown to comprise pairs of neighboring support arms 81.1, 81.2. All first and second neighboring support arms 81.1, 81.2 are combined by combining means 90 at their proximal end parts 81a to form united end parts 88a, so that all pairs of neighboring support arms 81.1, 81.2 are united at their proximal end parts 81a.

Further, all second and first neighboring support arms 81.2, 81.1 are combined by combining means 90 at their distal end parts 81b, to form united end parts 88b, such that all pairs of neighboring support arms 81.2, 81.1 are united at their distal end parts 81b. As shown in Fig. 3a, each support arm 81 is combined at its distal end part 81b with a neighboring support arm 81 that is different from the neighboring support arm to which it is combined or attached at its proximal end part 81a, which forms a mechanically stabilized framework that is configured to prevent or impede the undesired clustering of electrodes in a few locations along the patient's cardiac wall. Instead, the stabilized framework of support arms results in electrodes being much more evenly and regularly spaced and positioned along the patient's cardiac wall during an EP mapping procedure.

As shown in the Figures, an alteration in combining neighboring support arms 81.1, 81.2 may be employed, which besides forming united end parts 88a, 88b, further stabilizes the basket structure 83 of the electrode assembly 80. As a result, the central part 81c of an individual support arm 81 has a limited maximum angle β under which it may be deflected or bent towards a neighboring support arm when touching a surface. In one embodiment, this maximum possible angle β of deflection / freedom to be bent is less than ±((360°/x)-(360°/10)), preferably approximately ±(360°/x)/2) (with x being the number of support arms 81). As a further result, in use of the medical device, the electrode distribution of the basket structure 83 is more uniform than in the prior art, where support arms tend to bunch or cluster. In one embodiment, united end portions 88a, 88b may be formed in basket-type electrode assemblies 80 having an even number (number divisible by 2) of support arms 81.

The combining means 90 are preferably arranged on the border between the respective proximal end parts 81a / distal end parts 81b and the central parts 81c. In case of an adhesive or welded combining means 90, the combining means 90 may extend between this border and the pole area along a part of the length of the neighboring proximal and/or distal end parts 81a, 81b of the support arms 81 or over the entire length of the neighboring proximal and/or distal end parts 81a, 81b.

In the embodiment of Figures 3a, 4a, 5a the combining means 90 are formed as clamping elements 91 or clamps. By means of the clamping elements 91 the distal end parts 81b and the proximal end parts 81a of neighboring support arms 81.1, 81.2 are clamped together in tight fit. Such clamping elements 91 may easily be applied to a basket type catheter even after the electrode assembly 80 has been mounted at the elongated medical device 1. A retrofitting of basket type catheters with combining means 90 to realize the present invention is accordingly possible and such retrofitted catheters are also within the scope of the present invention and of what is described and disclosed herein.

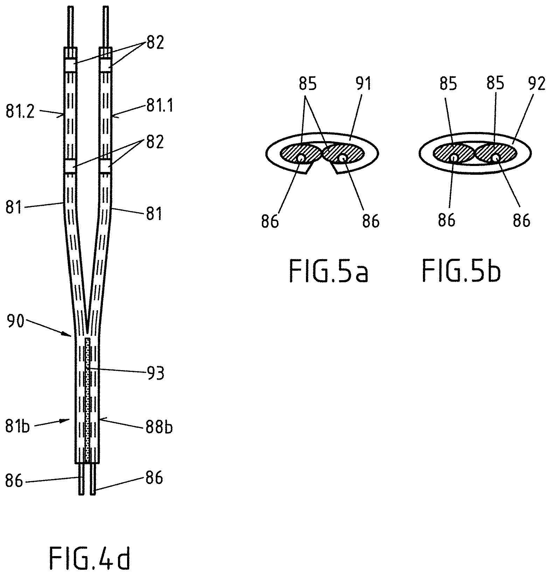

Some embodiments of alternative combining means 91 are displayed in Figures 4d and 5b. Many other types of combining structures and/or means are contemplated, however, including covers, sheaths, overmoldings, tubing, shrink tubing, clamps, ring members, rings, adhesives, lugs, welds, stakes, crimps, other suitable means or structures, or any combination or plurality thereof.

In the embodiment shown in Fig. 4d, the distal end parts 81b of neighboring support arms 81.1, 81.2 and the proximal end parts 81a of neighboring support arms 81.1, 81.2 are combined to form united end parts 88a, 88b by means of an adhesive joint 93. The adhesive joint 93 may be applied to portions or over almost the entire length of the united end parts 88a, 88b. Alternatively, a welded joint or welded connection may also be used to form joint 93. In the embodiment shown in Fig. 5b, the distal end parts 81b of neighboring support arms 81.1, 81.2 and the proximal end parts 81a of neighboring support arms 81.1, 81.2 are combined to united end parts 88a, 88b by means of ring members 92 or rings instead of clamping elements. Clamping means of different types may also be used in combination.

Further, instead or alternatively to a ring member 92 a tube or shrink tube (not shown in the Figures), e.g. made of a polymeric material, may be used which may encompass the united proximal and distal end parts 81a, 81b over a part of their length or over their entire length and combine them to form the united end parts 88a, 88b.

Combining means 90, especially in embodiments where clamps or clamping elements 91 or ring members 92, may also function as mapping electrodes. The combining means 90, formed of an electrically conductive metal, is electrically connected to electrical conductors or lines disposed in or on the support arms 81. The number of mapping electrodes may accordingly be augmented or enhanced.

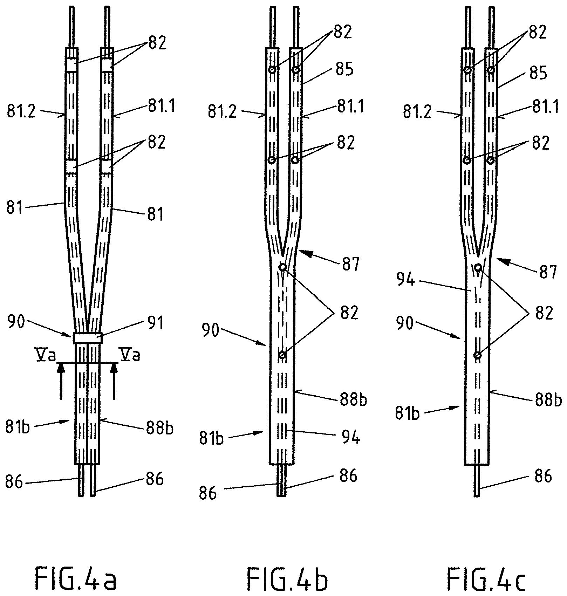

Referring again to Fig. 4a, in one embodiment each of support arms 81 comprises a strand 86 formed of a shape memory metal and a PCB (printed circuit board) layer 85, where the PCB layers 85 carry the electrodes 82 and electrical conductors (not visible in the Figures) for operative connection to electrodes 82. The PCB layers 85 at least partially surround the strands 86, which in one embodiment may be formed as Nitinol wires of 0.1 - 0.3 mm diameter, preferentially 0.2 mm diameter. Other suitable metals or metal alloys may also be employed to form such strands, wires or electrical conductors.

Referring again to Figures 2 and 3a, the electrode assembly 80 is connected via connection 12 with a data processing and control unit 15, which energizes and controls the electrodes 82. Data processing and control unit 15 processes electrode mapping data from the electrode assembly 80 and outputs mapping data on a data output screen 14 of a data output unit 16. Connection 12 may be a cable, ribbon cable, flat conductor, flat flexible cable or any other suitable electrical connection. At the end of connection 12 or line there are connectors 13 located for connecting the elongated medical device 1 and its electrode assembly 80 and associated electronics to the data processing and control unit 15.

In one embodiment, the data processing and control unit 15 may comprise a suitably programmed and configured computing device such as a personal computer. Elongated medical device 1 may form a portion of a catheter system interfaced to a computing device. In respect to the electrophysiological mapping data, in one embodiment the data processing and control unit 15 is configured to process analog and/or digitized electrode measurement data and to output data for visualizing circular excitation pattern (rotors), *e.g.,* in the left atrium of a patient's heart on a data output screen 14 of a data output unit 16.

In operation of the medical device 1, the medical device 1 or catheter is inserted in the patient's vessel, organ or other body cavity until it reaches the target area, which may be the left atrium of the heart, or any other portion of the heart. Upon arrival in the target area the operator may expand the electrode assembly 80 by manipulating the handle 7. In this expanded condition EC of the electrode assembly 80 and its support arms 81 the medical device 1 will be pushed with its distal end 4 against body tissue and electro-anatomic mapping may be initiated automatically or by the health care provider. Data analysis of electrophysiological data, such as action potential data, is performed on the data processing and control unit 15 respectively on a suitably programmed computing device.

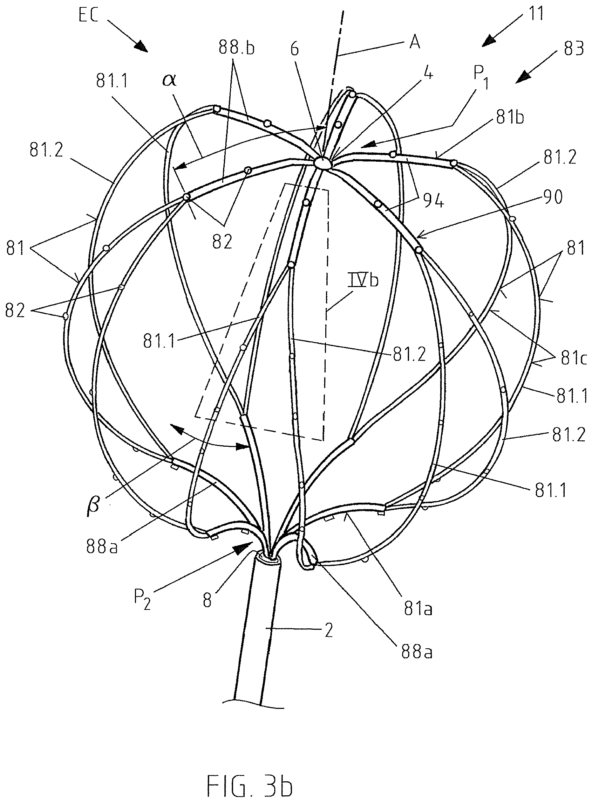

Figures 3b and 4b display a further embodiment of the elongated medical device 11. For reference numerals and functions not described in the following text, reference is made to the descriptions of Figs. 2, 3a, 4a and 5a. Similar parts are indicated with similar reference numerals. The embodiments shown in Figures 3b and 4b differ from those described with respect to Figs. 2, 3a, 4a and 5a in that the combining means 90 are realized in the form of a flexible electrical polymeric substrate or sheet 94 disposed at the united end parts 88a, 88b of the neighboring support arms 81.1, 81.2. Flexible electrical polymeric substrate or sheet 94 comprises electrical conductors, which in one embodiment are made using thin film electrical conductor deposition techniques. In some embodiments, the thin film electrical conductors and their corresponding electrodes may be formed or deposited on a material chosen from the group of Mylar, Kevlar, polyimide, PEEK, an electrically conductive polyester, or other suitable flexible biocompatible materials. Flexible electrical polymeric substrate or sheet 94 may also, *e.g.,* be formed as a PCB layer or an electro-ceramic layer. Further, and in the embodiments shown in Figs. 3b and 4b, two electrodes 82 are disposed on each united end part 88a, 88b of the support arms 81, while four electrodes 82 are located on each central parts 81c of the support arms 81.

The angle α defining the circumferential distance between the respective pole P1 or P2 and the border between the respective proximal end parts 81a / distal end parts 81b and the central parts 81c on this curve or bow is about 40° in this embodiment. Accordingly, a split point 87 (indicated in Fig. 4b) which lies in the border between the respective proximal end parts 81a / distal end parts 81b and the central parts 81c is in the same circumferential distance as defined by angle α of about 40°. Another way of saying this is that the angle α defines an angle swept substantially at a right angle to basket axis A between the respective proximal or distal end parts and the central parts of neighboring support arms.

Figure 4c displays a further embodiment of the elongated medical device similar to the embodiment of Figures 3b and 4b, but with modified support arms 81. For reference numerals and functions not described in the following text, reference is made to the descriptions of Figs. 2, 3a, 3b, 4a, 4b and 5a. Similar parts are indicated with similar reference numerals. The embodiment of Fig. 4c differs from that of Figures 3b and 4b in that there is only one shape memory strand 86 in the united end parts 88a, 88b, whereas there are two strands 86 in the embodiment of Figures 3b and 4b.

At the split point 87 the single strand 86 splits into two strands 86, one in each neighboring support arm 81.1, 81.2. Single strand 86 may be welded in split point 87 with the two strands 86 of the neighboring support arms 81.1, 81.2.

Note that the various embodiments include those described above in the Summary, where, for example, the attachment of distal and proximal end parts in neighboring support arms is reversed, one end of a support arm need not be attached to a neighboring support arm (but only to a support arm that is not the same as that to which the support arm is attached or combined at one end).

The various systems, devices, components and methods described and disclosed herein may also be adapted and configured for use in electrophysiological mapping applications other than those involving the interior of a patient's heart. These alternative applications include EP mapping and diagnosis of a patient's epicardium, a patient's spinal cord or other nerves, or a patient's brain or portions thereof.

In addition, the elongated medical device or cardiac mapping catheter described and disclosed herein may be modified to include a cardiac ablation device at or near distal end 4 or tip 6. Such ablation devices may include, but are not limited to RF, cryogenic, and radioactive ablation devices. The cardiac mapping catheter may also include a force sensor, temperature sensor and/or irrigation device disposed near or at its tip 6. Moreover, the various embodiments described and disclosed herein include methods of making and using same, as described, for example, in the Summary above.

What have been described above are examples and embodiments of the devices and methods described and disclosed herein. It is, of course, not possible to describe every conceivable combination of components or methodologies for purposes of describing the invention, but one of ordinary skill in the art will recognize that many further combinations and permutations of the devices and methods described and disclosed herein are possible. Accordingly, the devices and methods described and disclosed herein are intended to embrace all such alterations, modifications and variations that fall within the scope of the appended claims. In the claims, unless otherwise indicated, the article "a" is to refer to "one or more than one."

After having read and understood the present specification, those skilled in the art will now understand and appreciate that the various embodiments described herein provide solutions to long-standing problems, both in the use of electrophysiological mapping systems and in the use of cardiac ablation systems.

### Reference list

- 1: elongated medical device
- 2: elongated body
- 3: distal portion
- 4: distal end
- 5: proximal portion
- 6: tip
- 7: handle
- 8: space

- 11: further elongated medical device
- 12: connection
- 13: connectors
- 14: data output screen
- 15: data processing and control unit
- 16: data output unit

- 80: electrode assembly/mapping electrode assembly
- 81: support arms
- 81a: proximal end part
- 81b: distal end part
- 81c: main part
- 81.1: first support arm
- 81.2: second support arm
- 82: electrodes/mapping electrodes
- 83: basket structure

- 85: flexible electrical polymeric sheets / PCB layers
- 86: (shape memory metal) strands
- 87: split point
- 88a: united or combined end parts
- 88b: united or combined end parts

- 90: combining means
- 91: clamping element
- 92: ring member
- 93: adhesive joint
- 94: common flexible electrical polymeric sheet / PCB layer

- 110: elongated medical device (Prior Art)
- 112: elongated body
- 113: distal portion
- 114: distal end

- 116: tip

- 180: electrode assembly/mapping electrode assembly
- 181: support arms
- 182: electrodes/mapping electrodes

- 190: cluster

- A: basket axis
- EC: second (expanded) condition (of 80)
- P1: first pole area
- P2: second pole area
- S: surface
- UC: first (retracted) condition (of 80)
- α: angle (between the pole areas and the respective border between the proximal / distal end parts and the central parts of the support arms)
- β: maximum possible angle of deflection of the central parts of the support arms

## Claims

1. An elongated medical device (1, 11) for electro-anatomic mapping and suitable for intravascular insertion, said device comprising
a flexible elongated body (2) having a distal portion (3) with a distal end (4) and a proximal portion (5), and
an electrode assembly (80) located at the distal portion (3), the electrode assembly (80) comprising
- a plurality of at least x=4 support arms (81), wherein x is evenly divisible by the number 2, each support arm (81) having a proximal end part (81a), a distal end part (81b) and a main part (81c) between the proximal end part (81a) and the distal end part (81b), the plurality of at least x=4 support arms (81) comprising first and second neighboring support arms (81.1, 81.2) forming pairs of neighboring support arms (81), and
- a plurality of electrodes (82) arranged on the support arms (81) for collecting electrophysiological data,
the plurality of at least x=4 support arms (81) being configured to have a first condition (UC), where the plurality of at least x=4 support arms (81) is arranged in a bundle, and a second condition (EC), where the plurality of at least x=4 support arms (81) is expanded, forming a basket type structure (83),
**characterized in that**,
each first and second neighboring support arms (81.1, 81.2) are combined at their proximal end parts (81a) to form united end parts (88a), such that all pairs of neighboring support arms (81) are united at their proximal end parts (81a), and that
each second and first neighboring support arms (81.2, 81.1) are combined at their distal end parts (81b), to form united end parts (88b), such that all pairs of neighboring support arms (81) are united at their distal end parts (81b), and wherein each support arm (81) is combined at its distal end part (81b) with a different neighboring support arm (81) than at its proximal end part (81a).

2. Medical device (1, 11) according to claim 1, **characterized in that** the combination of the proximal end parts (81a) and/or the distal end parts (81b) of neighboring support arms (81) is realized by combining means (90) that are disposed at each united end part (88a, 88b).

3. Medical device (1, 11) according to claim 2, **characterized in that** the combining means (90) comprises one or more of a clamping element (91), a ring member (92), an adhesive joint (93), and a lug or any combination or plurality thereof

4. Medical device (1, 11) according to any of claims 1 to 3, **characterized in that** each support arm (81) comprises a flexible electrical polymeric sheet (85) that carries the electrodes (82), wherein the united end parts (88a, 88b) of the support arms (81) share a common flexible electrical polymeric sheet (94).

5. Medical device (1, 11) according to any of the preceding claims, **characterized in that** the number x of support arms (81) equals 4, 6, 8, 10 or 12.

6. Medical device (1, 11) according to any of the preceding claims, **characterized in that** the plurality of electrodes (82) comprises a number of 4 to 24 electrodes disposed on each of the support arms (81)

7. Medical device (1, 11) according to any of the preceding claims, **characterized in that** the plurality of electrodes (82) is evenly distributed on the support arms (81).

8. Medical device (1, 11) according to any of the preceding claims, **characterized in that** the basket type structure (83) has two pole areas (P1, P2) that lie in a basket axis (A), wherein in the second condition (EC) each of the support arms (81) span a curve of about 180° between the two pole areas (P1, P2), and wherein an angle (α) defines the circumferential distance between the respective pole area (P1, P2) and a border between the respective proximal or distal end parts (81a, 81b) and the central parts (81c) on this curve, and wherein the angle (α) is in a range of 5° to 40°.

9. Medical device (1, 11) according to claim 8, **characterized in that** the angle (α) is in the range of 5° to 25°.

10. Medical device (1, 11) according to any of the preceding claims, **characterized in that** the combining means (90) are formed of a conductive metal and are electrically connected to electric lines of the support arms (81) such that the combining means (90) function as mapping electrodes.

11. Medical device (1, 11) according to any of the preceding claims, **characterized in that** it is formed as a catheter for exploration or treatment of a vessel, organ or other body cavity.

12. A method of making an elongated medical device (1, 11) for electro-anatomic mapping and suitable for intravascular insertion, the method comprising:
forming a flexible elongated body (2) having a distal portion (3) with a distal end (4) and a proximal portion (5), and
forming an electrode assembly (80) configured to be located at or near the distal portion (3), the electrode assembly (80) comprising a plurality x of at least 4 support arms (81), wherein x is evenly divisible by the number 2, each support arm (81) having a proximal end part (81a), a distal end part (81b), and a main part (81c) located between the proximal end part (81a) and the distal end part (81b), each of the plurality of support arms (81) comprising a plurality of electrodes (82), the electrodes (82) being configured to acquire electrophysiological signals, the plurality of support arms (81) being configured to have a first retracted condition (UC) where the plurality of support arms (81) is arranged in a collapsed bundle, and a second expanded condition (EC) where the plurality of support arms (81) forms an expanded basket structure (83);
wherein the plurality of support arms (81) further comprises first and second neighboring support arms (81.1, 81.2),
**characterized in that** the proximal end parts (81a) of the first and second neighboring support arms (81.1, 81.2) being combined or held together near or adjoining one another, the distal end part (81b) of the first support arm (81.1) being combined or held together near or adjoining a distal end part (81b) of a neighboring support arm (81) that is not the second support arm (81.2), the distal end part (81b) of the second support arm (81.2) being combined or held together near or adjoining a distal end part (81b) of a neighboring support arm (81) that is not the first support arm (81.1), each of the plurality of support arms (81) in the electrode assembly being combined or held together at or near its distal end part (81b) with a neighboring support arm (81) that is different from the neighboring support arm (81) with which it is combined or held together at its proximal end part (81a).

13. The method according to claim 12, further comprising combining or holding together the distal end parts (81b) or proximal end parts (81a) with combining means (90).

14. The method according to claim 12 or 13, wherein the combining means (90) comprises one or more of a clamping element (91), a ring member (92), an adhesive joint (93), a lug, or any combination or plurality thereof.

15. The method according to any of claims 12 to 13, wherein the basket type structure (83) if formed to have two pole areas (P1, P2) that lie in a basket axis (A), wherein in the second condition (EC) each of the support arms (81) span a curve of about 180° between the two pole areas (P1, P2), and wherein an angle (α) defines the circumferential distance between the respective pole area (P1, P2) and a border between the respective proximal or distal end parts (81a, 81b) and the central parts (81c) on this curve, and wherein the angle (α) is in a range of 5° to 40°.

## Patentansprüche

1. Langgestreckte medizinische Vorrichtung (1, 11) für eine elektroanatomische Abbildung, die für eine intravaskuläre Einführung geeignet ist, wobei die Vorrichtung umfasst:
einen flexiblen langgestreckten Körper (2), der einen distalen Teil (3) mit einem distalen Ende (4) und einen proximalen Teil (5) besitzt, und
eine Elektrodenanordnung (80), die sich an dem distalen Teil (3) befindet, wobei die Elektrodenanordnung (80) umfasst:
- eine Mehrzahl von mindestens x = 4 Tragarmen (81), wobei x geradzahlig ist, wobei jeder Tragarm (81) einen proximalen Endteil (81a), einen distalen Endteil (81b) und einen Hauptteil (81c) zwischen dem proximalen Endteil (81a) und dem distalen Endteil (81b) besitzt, wobei die Mehrzahl von mindestens x = 4 Tragarmen (81) erste und zweite benachbarte Tragarme (81.1, 81.2), die Paare benachbarter Tragarme (81) bilden, besitzt, und
- mehrere Elektroden (82), die auf den Tragarmen (81) zum Sammeln elektrophysiologischer Daten angeordnet sind,
wobei die Mehrzahl von mindestens x = 4 Tragarmen (81) konfiguriert ist, einen ersten Zustand (UC), in dem die Mehrzahl von mindestens x = 4 Tragarmen (81) in einem Bündel angeordnet ist, und einen zweiten Zustand (EC), in dem die Mehrzahl von mindestens x = 4 Tragarmen (81) ausgebreitet ist und eine Korbtypstruktur (83) bildet, zu besitzen,
**dadurch gekennzeichnet, dass**
jeder erste und zweite benachbarte Tragarm (81.1, 81.2) an ihren proximalen Endteilen (81a) vereinigt sind, um vereinigte Endteile (88a) zu bilden, so dass alle Paare benachbarter Tragarme (81) an ihren proximalen Endteilen (81a) vereinigt sind,
und dass
jeder zweite und erste benachbarte Tragarm (81.2, 81.1) an ihren distalen Endteilen (81b) vereinigt sind, um vereinigte Endteile (88b) zu bilden, so dass alle Paare benachbarter Tragarme (81) an ihren distalen Endteilen (81b) vereinigt sind,
wobei jeder Tragarm (81) an seinem distalen Endteil (81b) mit einem anderen benachbarten Tragarm (81) als an seinem proximalen Endteil (81a) vereinigt ist.

2. Medizinische Vorrichtung (1, 11) nach Anspruch 1, **dadurch gekennzeichnet, dass** die Vereinigung der proximalen Endteile (81a) und/oder der distalen Endteile (81b) benachbarter Tragarme (81) durch Vereinigungsmittel (90) realisiert wird, die an jedem vereinigten Endteil (88a, 88b) angeordnet sind.

3. Medizinische Vorrichtung (1, 11) nach Anspruch 2, **dadurch gekennzeichnet, dass** die Vereinigungsmittel (90) ein oder mehrere Klemmelemente (91), ein Ringelement (92), eine Klebverbindung (93) und eine Lasche oder eine Kombination oder mehrere davon umfassen.

4. Medizinische Vorrichtung (1, 11) nach einem der Ansprüche 1 bis 3, **dadurch gekennzeichnet, dass** jeder Tragarm (81) eine flexible elektrische polymere Folie (85) umfasst, die die Elektroden (82) trägt, wobei die vereinigten Endteile (88a, 88b) der Tragarme (81) eine gemeinsame flexible elektrische polymere Folie (94) gemeinsam nutzen.

5. Medizinische Vorrichtung (1, 11) nach einem der vorhergehenden Ansprüche, **dadurch gekennzeichnet, dass** die Anzahl x von Tragarmen (81) gleich 4, 6, 8, 10 oder 12 ist.

6. Medizinische Vorrichtung (1, 11) nach einem der vorhergehenden Ansprüche, **dadurch gekennzeichnet, dass** die mehreren Elektroden (82) eine Anzahl von 4 bis 24 Elektroden umfassen, die an jedem der Tragarme (81) angeordnet sind.

7. Medizinische Vorrichtung (1, 11) nach einem der vorhergehenden Ansprüche, **dadurch gekennzeichnet, dass** die mehreren Elektroden (82) auf dem Tragarmen (81) gleichmäßig verteilt sind.

8. Medizinische Vorrichtung (1, 11) nach einem der vorhergehenden Ansprüche, **dadurch gekennzeichnet, dass** die Korbtypstruktur (83) zwei Polflächen (P1, P2) besitzt, die in einer Korbachse (A) liegen, wobei in dem zweiten Zustand (EC) jeder der Tragarme (81) eine Kurve von ungefähr 180° zwischen den beiden Polflächen (P1, P2) überspannt und wobei ein Winkel (a) den Umfangsabstand zwischen den jeweiligen Polflächen (P1, P2) und einer Grenze zwischen den jeweiligen proximalen oder distalen Endteilen (81a, 81b) und den mittleren Teilen (81c) auf dieser Kurve definiert und wobei der Winkel (a) in einem Bereich von 5° bis 40° liegt.

9. Medizinische Vorrichtung (1, 11) nach Anspruch 8, **dadurch gekennzeichnet, dass** der Winkel (a) im Bereich von 5° bis 25° liegt.

10. Medizinische Vorrichtung (1, 11) nach einem der vorhergehenden Ansprüche, **dadurch gekennzeichnet, dass** die Vereinigungsmittel (90) aus einem leitenden Metall gebildet sind und mit elektrischen Leitungen der Tragarme (81) derart elektrisch verbunden sind, dass die Vereinigungsmittel (90) als Abbildungselektroden arbeiten.

11. Medizinische Vorrichtung (1, 11) nach einem der vorhergehenden Ansprüche, **dadurch gekennzeichnet, dass** sie als ein Katheter für eine Erforschung oder Behandlung eines Blutgefäßes, eines Organs oder eines anderen Körperhohlraums gebildet ist.

12. Verfahren zum Herstellen einer langgestreckten medizinischen Vorrichtung (1, 11) für eine elektroanatomische Abbildung, die für eine intravaskuläre Einführung geeignet ist, wobei das Verfahren umfasst:
Bilden eines flexiblen langgestreckten Körpers (2), der einen distalen Teil (3) mit einem distalen Ende (4) und einen proximalen Teil (5) besitzt, und
Bilden einer Elektrodenanordnung (80), die so konfiguriert ist, dass sie sich an dem distalen Teil (3) oder in dessen Nähe befindet, wobei die Elektrodenanordnung (80) eine Mehrzahl von mindestens x = 4 Tragarmen (81) umfasst, wobei x geradzahlig ist, wobei jeder Tragarm (81) einen proximalen Endteil (81a), einen distalen Endteil (81b) und einen Hauptteil (81c), der sich zwischen dem proximalen Endteil (81a) und dem distalen Endteil (81b) befindet, besitzt, wobei jeder der mehreren Tragarme (81) mehrere Elektroden (82) umfasst, wobei die Elektroden (82) konfiguriert sind, elektrophysiologische Signale zu erfassen, wobei die mehreren Tragarme (81) so konfiguriert sind, dass sie einen ersten zurückgezogenen Zustand (UC), in dem die mehreren Tragarme (81) in einem zusammengeklappten Bündel angeordnet sind, und einen zweiten ausgebreiteten Zustand (EC), in dem die mehreren Tragarme (81) eine ausgedehnte Korbtypstruktur (83) bilden, besitzen,
wobei die mehreren Tragarme (81) ferner erste und zweite benachbarte Tragarme (81.1, 81.2) umfassen,
**dadurch gekennzeichnet, dass** die proximalen Endteilen (81a) der ersten und zweiten benachbarten Tragarme (81.1, 81.2) nah oder aneinander angrenzend vereinigt sind oder zusammengehalten werden, wobei der distale Endteil (81b) des ersten Tragarmes (81.1) nah an einem distalen Endteil (81b) eines benachbarten Tragarms (81), der nicht der zweite Tragarm (81.2) ist, oder an ihn angrenzend vereinigt ist oder zusammengehalten wird, wobei der distale Endteil (81b) des zweiten Tragarms (81.2) nah an einem distalen Endteil (81b) eines benachbarten Tragarms (81), der nicht der erste Tragarm (81.1) ist, oder an ihn angrenzend vereinigt ist oder zusammengehalten wird, wobei jeder der mehreren Tragarme (81) in der Elektrodenanordnung an seinem distalen Endteil (81b) oder in dessen Nähe mit einem benachbarten Tragarm (81), der von dem benachbarten Tragarm (81) verschieden ist, mit dem er an seinem proximalen Endteil (81a) vereinigt ist oder zusammengehalten wird, vereinigt ist oder zusammengehalten wird.

13. Verfahren nach Anspruch 12, das ferner umfasst, die distalen Endteile (81b) oder proximalen Endteile (81a) mit Vereinigungsmitteln zu vereinigen oder zusammenzuhalten.

14. Verfahren nach Anspruch 12 oder 13, wobei die Vereinigungsmittel (90) ein oder mehrere Klemmelemente (91), ein Ringelement (92), eine Klebverbindung (93) und eine Lasche oder eine Kombination oder mehrere davon umfassen.

15. Verfahren nach einem der Ansprüche 12 bis 13, wobei die Korbtypstruktur (83) so gebildet ist, dass sie zwei Polflächen (P1, P2) besitzt, die in einer Korbachse (A) liegen, wobei in dem zweiten Zustand (EC) jeder der Tragarme (81) eine Kurve von ungefähr 180° zwischen den beiden Polflächen (P1, P2) überspannt und wobei ein Winkel (a) den Umfangsabstand zwischen den jeweiligen Polflächen (P1, P2) und einer Grenze zwischen den jeweiligen proximalen oder distalen Endteilen (81a, 81b) und den mittleren Teilen (81c) auf dieser Kurve definiert und wobei der Winkel in einem Bereich von 5° bis 40° liegt.

## Revendications

1. Dispositif médical allongé (1, 11) pour la cartographie électro-anatomique et conçu pour insertion intravasculaire, ledit dispositif comprenant
un corps allongé flexible (2) ayant une partie distale (3) avec une extrémité distale (4) et une partie proximale (5), et
un ensemble d'électrode (80) situé à la partie distale (3), l'ensemble d'électrode (80) comprenant
une pluralité d'au moins x=4 bras de support (81), dans laquelle x est divisible de manière égale par le nombre 2, chaque bras de support (81) ayant une partie d'extrémité proximale (81a), une partie d'extrémité distale (81b) et une partie principale (81c) entre la partie d'extrémité proximale (81a) et la partie d'extrémité distale (81b), la pluralité d'au moins x=4 bras de support (81) comprenant des premier et second bras de support voisins (81.1, 81.2) formant des paires de bras de support voisins (81), et
une pluralité d'électrodes (82) disposées sur les bras de support (81) pour la collecte de données électrophysiologiques,
la pluralité d'au moins x=4 bras de support (81) étant configurée pour avoir une première condition (UC), où la pluralité d'au moins x=4 bras de support (81) est disposée en un faisceau, et une second condition (EC), où la pluralité d'au moins x=4 bras de support (81) est étendue, formant une structure de type panier (83),
**caractérisé en ce que**,
chaque premier et second bras de support voisin (81.1, 81.2) sont combinés à leurs parties d'extrémité proximale (81a) pour former des parties d'extrémité unies (88a), de sorte que toutes les paires de bras de support voisins (81) sont unies à leurs parties d'extrémité proximale (81a), et que
chaque second et premier bras de support voisin (81.2, 81.1) sont combinés à leurs parties d'extrémité distale (81b), pour former des parties d'extrémité unies (88b), de sorte que toutes les paires de bras de support voisins (81) sont unies à leurs parties d'extrémité distale (81b), et
dans lequel chaque bras de support (81) est combiné à sa partie d'extrémité distale (81b) avec un bras de support voisin (81) différent de celui de sa partie d'extrémité proximale (81a).

2. Dispositif médical (1, 11) selon la revendication 1, **caractérisé en ce que** la combinaison des parties d'extrémité proximales (81a) et/ou des parties d'extrémité distales (81b) des bras de support voisins (81) est réalisée par des moyens de combinaison (90) qui sont disposés à chaque partie d'extrémité unie (88a, 88b).

3. Dispositif médical (1, 11) selon la revendication 2, **caractérisé en ce que** le moyen de combinaison (90) comprend un ou plusieurs éléments parmi un élément de serrage (91), un élément annulaire (92), un joint adhésif (93) et une patte ou toute combinaison ou pluralité de ceux-ci.

4. Dispositif médical (1, 11) selon l'une des revendications 1 à 3, **caractérisé en ce que** chaque bras de support (81) comprend une feuille polymère électrique flexible (85) qui porte les électrodes (82), dans lequel les parties d'extrémité unies (88a, 88b) des bras de support (81) partagent une feuille polymère électrique flexible commune (94).

5. Dispositif médical (1, 11) selon l'une des revendications précédentes, **caractérisé en ce que** le nombre x de bras de support (81) est égal à 4, 6, 8, 10 ou 12.

6. Dispositif médical (1, 11) selon l'une quelconque des revendications précédentes, **caractérisé en ce que** la pluralité d'électrodes (82) comprend un nombre de 4 à 24 électrodes disposées sur chacun des bras de support (81)

7. Dispositif médical (1, 11) selon l'une des revendications précédentes, **caractérisé en ce que** la pluralité d'électrodes (82) est répartie uniformément sur les bras de support (81).

8. Dispositif médical (1, 11) selon l'une des revendications précédentes, **caractérisé en ce que** la structure de type panier (83) a deux zones polaires (P1, P2) qui se trouvent dans un axe de panier (A), dans lequel, dans la second condition (CE), chacun des bras de support (81) couvre une courbe d'environ 180° entre les deux zones polaires (P1, P2), et dans lequel un angle (a) définit la distance circonférentielle entre la zone polaire respective (PI, P2) et une frontière entre les parties d'extrémité proximale ou distale respectives (81a, 81b) et les parties centrales (81c) sur cette courbe, et dans lequel l'angle (a) est dans une plage de 5° à 40°.

9. Dispositif médical (1, 11) selon la revendication 8, **caractérisé en ce que** l'angle (a) se situe dans la plage de 5° à 25°.

10. Dispositif médical (1, 11) selon l'une des revendications précédentes, **caractérisé en ce que** les moyens de combinaison (90) sont formés d'un métal conducteur et sont électriquement connectés à des lignes électriques des bras de support (81) de telle sorte que les moyens de combinaison (90) fonctionnent comme des électrodes de cartographie.

11. Dispositif médical (1, 11) selon l'une des revendications précédentes, **caractérisé en ce qu'**il est formé comme un cathéter pour l'exploration ou le traitement d'un vaisseau, d'un organe ou d'une autre cavité corporelle.

12. Procédé de fabrication d'un dispositif médical allongé (1, 11) pour la cartographie électro-anatomique et conçu pour insertion intravasculaire, le procédé comprenant:
la formation d'un corps allongé flexible (2) ayant une partie distale (3) avec une extrémité distale (4) et une partie proximale (5), et
la formation d'un ensemble d'électrodes (80) configuré pour être situé au niveau ou à proximité de la partie distale (3), l'ensemble d'électrodes (80) comprenant une pluralité x d'au moins 4 bras de support (81), où x est divisible de manière égale par le nombre 2, chaque bras de support (81) ayant une partie d'extrémité proximale (81a), une partie d'extrémité distale (81b) et une partie principale (81c) située entre la partie d'extrémité proximale (81a) et la partie d'extrémité distale (81b), chacun de la pluralité de bras de support (81) comprenant une pluralité d'électrodes (82), les électrodes (82) étant configurées pour acquérir des signaux électrophysiologiques, la pluralité de bras de support (81) étant configurée pour avoir un premier mode rétracté (UC) dans lequel la pluralité de bras de support (81) est disposée en faisceau replié, et un second mode élargi (CE) où la pluralité des bras de soutien (81) forme une structure de panier élargie (83);
dans lequel la pluralité de bras de support (81) comprend en outre un premier et un second bras de support voisins (81.1, 81.2),
**caractérisé en ce que** les parties d'extrémité proximale (81a) des premier et second bras de support voisins (81.1, 81.2) sont combinées ou maintenues ensemble près ou en contact l'une avec l'autre, la partie d'extrémité distale (81b) du premier bras de support (81.1) étant combinée ou maintenue ensemble près ou en contact avec une partie d'extrémité distale (81b) d'un bras de support voisin (81) qui n'est pas le second bras de support (81.2), la partie d'extrémité distale (81b) du second bras de support (81.2) étant combinée ou maintenue ensemble près ou contiguë à une partie d'extrémité distale (81b) d'un bras de support voisin (81) qui n'est pas le premier bras de support (81.1), chacun de la pluralité de bras de support (81) dans l'ensemble d'électrodes étant combiné ou maintenu ensemble au niveau ou près de sa partie d'extrémité distale (81b) avec un bras de support voisin (81) qui est différent du bras de support voisin (81) avec lequel il est combiné ou maintenu ensemble au niveau de sa partie d'extrémité proximale (81a).

13. Procédé selon la revendication 12, comprenant en outre la combinaison ou le maintien ensemble des parties d'extrémité distale (81b) ou des parties d'extrémité proximale (81a) avec des moyens de combinaison (90).

14. Procédé selon les revendications 12 ou 13, dans lequel le moyen de combinaison (90) comprend un ou plusieurs éléments parmi un élément de serrage (91), un élément annulaire (92), un joint adhésif (93), une patte, ou toute combinaison ou pluralité de ceux-ci.

15. Procédé selon l'une quelconque des revendications 12 à 13, dans lequel la structure de type panier (83) est formée pour avoir deux zones polaires (PI, P2) qui se trouvent dans un axe de panier (A), dans lequel, dans la seconde condition (EC), chacun des bras de support (81) couvre une courbe d'environ 180° entre les deux zones polaires (PI, P2), et dans lequel un angle (a) définit la distance circonférentielle entre la zone polaire respective (P1, P2), et une frontière entre les parties d'extrémité proximale ou distale respectives (81a, 81b) et les parties centrales (81c) sur cette courbe, et dans lequel l'angle (a) est dans une plage de 5° à 40°.
